(19) **Europäisches Patentamt European Patent Office Office européen des brevets**

(11) **EP 4 714 470 A1**

(12) **EUROPEAN PATENT APPLICATION**

(43) Date of publication:
**25.03.2026 Bulletin 2026/13**

(21) Application number: **24306541.4**

(22) Date of filing: **19.09.2024**

(51) International Patent Classification (IPC):
*A61L 2/14* (2006.01) *H05H 1/46* (2006.01)

(52) Cooperative Patent Classification (CPC):
**A61L 2/14; H01J 37/321; H01J 37/32146;**
A61L 2103/15; A61L 2202/122

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
 • **Aurora**
   **76000 Rouen (FR)**
 • **Université De Reims Champagne-Ardenne**
   **51100 Reims (FR)**

(72) Inventors:
 • **PARIAS, Thomas**
   **76000 Rouen (FR)**
 • **LE BRAS, Florian**
   **76000 Rouen (FR)**
 • **JUDEÉ, Florian**
   **76000 Rouen (FR)**
 • **CHARMOT, Alexandre**
   **76000 Rouen (FR)**
 • **VILLEGER, Sandrine**
   **76000 Rouen (FR)**
 • **GELLÉ, Marie-Paule**
   **51724 Reims Cedex (FR)**

(74) Representative: **Icosa**
   **83 avenue Denfert-Rochereau**
   **75014 Paris (FR)**

(54) **DEVICE AND METHOD FOR STERILIZING OBJECTS**

(57)    The present invention relates to sterilization, and more particularly sterilization of medical devices.

**FIG. 1**

EP 4 714 470 A1

**Description**

**FIELD OF INVENTION**

The present invention relates to sterilization, and more particularly sterilization of medical devices.

**BACKGROUND OF INVENTION**

Sterilizing objects and devices are a crucial aspect for many applications, particularly in medical applications and/or in-vivo applications to avoid bacterial contamination, degradation and/or disease. In the wake of Covid, several sterilizing technologies have been developed, such as UV lights technologies. However, said technologies only shows reduction of bio contaminants levels, but often fail to reach the sterilization as defined in ISO 14937. According to ISO 14937, a process is considered a sterilizing process when, for an object sterilized by the process, the probability of finding contamination by the presence of a biological material (bacteria, viruses, fungus, or spores) on said object is inferior to one out of one million (i.e. a probability lower than $10^{-6}$). For instance, sterilizing an endoscope before an operation on a patient is mandatory, to avoid any risk of complication for the patient. However, sterilization is not an easy straight forward process. The challenge lies in the efficiency of the process against the most resistant strains. Moreover, some objects like endoscopes are complex to sterilize. Indeed, such objects are complex in shape, with difficult parts to reach, and comprise numerous materials, some of which being very sensitive that may present a risk of being damaged by the sterilization process, thus affecting the device function or generating toxic residues.

Among the reference practices, to reach a sterile state, the object to be sterilized may be brought under atmosphere comprising ozone. However, such a method is not able to sterilize organic materials such as cellulose, collagen or other resins and polymers, since it damages said organic material. Furthermore, ozone sterilization is little penetrative, thereby limiting the range of possible applications. Moreover, ozone is generally considered as a dangerous and harmful chemical for human being and therefore its use is banned in a growing number of hospitals. Due to risk of residues, its use for sterilization is not authorized for medical devices. Thus, medical applications are excluded.

Other sterilization methods, compatible with medical applications, are known. In particular, methods involving autoclave or hydrogen peroxide are common for medical applications. However, electronic devices and organic materials are damaged by said methods, which are therefore not recommended for sterilizing them. For instance, heat treatment which are either dry heat at 160°C to 190°C or wet heat at 134°C combined with pressure of 20 to 30 psi (the autoclave method,) have been known and used successfully to sterilize medical devices like metal surgical tools, glass containers and parts. However, with the development use of more complex materials like polymers and electronics, heat treatments reach some limitations: the excess heat may affect the object to be sterilized and either temper with its functionalities or result in premature degradation of polymers with the subsequent loss of mechanical properties, or the generation of harmful or toxic residues, or even deteriorate electronic components or connections. On the other hand, alternative methods requiring less heat which have been developed to address these types of issues generally relies on the use of chemicals like hydrogen peroxide, peracetic acid or ethylene oxide. Nonetheless, chemical methods remain limited in the treatment of complex shape as the chemicals do not penetrate complex shapes like long lumens in endoscopes like gastroscopes and above all may present significant health risk for a patient, because of the residues of the chemical vector which is in use and which are harmful for the body of a patient.

Other sterilization method, involving plasma, are known. However, igniting a plasma requires strict conditions that may not be compatible with the continuity of the sterilized state of an object. For instance, if an object is disposed inside a packaging, plasma may not be able to reach the inside of the packaging and achieve the sterilization of the said object, and on the other hand, if the object is removed from the packaging in order to be directly exposed to the reactive species generated by the plasma to be sterilized, the continuity of the sterile state can not be maintained as it would be recontaminated after the process while being taken by operators to be placed in its packaging.

Also, plasma ignition generally requires a strong energy input resulting in high current and high temperature. Such conditions may affect the devices integrity. This issue has been tentatively addressed by the development of the post discharge plasma treatment. Indeed, this setup suggest placing a plasma source away from the object to be sterilized, then flow the reactive species into a treatment chamber while the active species diffuse around the object to achieve a sterile state. However post-discharge method fails to provide a density of active species into the chamber high enough to preserve the sterile state post treatment. It also fails to achieve a satisfying treatment of complex shapes like long lumens in endoscopes.

Some sterilization technologies based on the use of chemicals such as hydrogen peroxide have been combined with the use of a plasma phase. It allows decomposing the toxic chemicals and reducing residue levels. However, this method still face the limitations of the diffusion of the chemical species which cannot treat complex shapes.

In November 2022, in Barcelona at the annual conference of the World Federation for Hospital Sterilisation Sciences, Dr Lionel Pineau from Eurofins presented a 7 years study on endoscopes contamination in France: *Endoscope*

*Reprocessing : Retrospective Analysis of 90311 Samples* - in which he reported alarming contamination levels of over 21% of endoscopes in hospitals due to the lack of proper decontamination procedure and especially sterilisation. Thus, there is a need for improvement of sterilization methods, in particular for sterilizing complex medical devices.

**SUMMARY**

This invention relates to a non-thermal plasma sterilizing device including:

a primary enclosure fluidly connected to a pumping system,
a secondary enclosure configured to receive an object to be sterilized, the secondary enclosure being configured to be removably disposed inside the primary enclosure and comprising an output port fluidly connected to the primary enclosure, the secondary enclosure being impermeable to biological materials and permeable to electromagnetic fields,
wherein the secondary enclosure encompasses a gas, the pumping system being configured to pump the gas encompassed in the secondary enclosure through the output port, and
wherein the sterilizing device further comprises an electromagnetic source configured to generate an electromagnetic field to ignite the gas of the secondary enclosure into a non-thermal plasma.

In the present description, a biological material is any type of organic living materials. For instance, viruses, spores, bacteria, cells, fungus, etc... are considered as biological materials.

In the present description, an object is considered sterilized when the probability of presence of a biological material on said object is inferior to $10^{-6}$. According to ISO 14937, it is measured by achieving the complete destruction of $10^6$ colony forming units (cfu) of the most resistant strain- i.e. a 6 logic reduction of a reference strain population.

In the present description, a non-thermal plasma is a gas plasma which is not in thermodynamic equilibrium. It is defined with the ionization coefficient

$$\alpha = \frac{n_i}{(n_i + n_n)}$$

where $n_n$ is the density of the gas in which the plasma is active, $n_i$ is the density of positively charged particles and $n_e$ is the electronic density. This coefficient varies from 1 for fully ionized plasma (very hot) down to $10^{-4}$ for low temperature plasma with a low ionization level. Such a low temperature plasma is also called a non-thermal plasma. The following table provides some information on properties of plasma.

| Parameters | Hot Plasma | Non Thermal Plasma |
|---|---|---|
| Temperature | Local Thermodynamic Equilibrium $T_e \cong T_i = T_n$ $\sim 10^4 K \sim 1 eV$ | No Local Thermodynamic Equilibrium $T_e >> T_i \approx T_n$ $T_e \sim 1 - 10 eV$ $T_i \sim 300K \sim 1000K$ |
| Density | $n_e >> 10^{13} cm^{-3}$ | $n_e \approx 10^6 - 10^{13} cm^{-3}$ |
| Ionization Level | High ionization level $\alpha \approx 10^{-2} - 1$ | Partial ionization level $\alpha \approx 10^{-5} - 10^{-2}$ |

In low temperature plasma only a small fraction of the electrons are activated and the fraction of the activated atoms remains small compared to the overall gas atoms typically in the range of $10^6 cm^{-3}$ to $10^{13} cm^{-3}$ more preferably in the range of $3.10^6 cm^{-3}$ to $3.10^8 cm^{-3}$. Hence the overall gas temperature (directly related to the speed of each atoms of molecule in the gas) remains low - here considered lower than 50°C. Non-thermal plasma requires an energy input to ignite and may, for instance, be electrically induced. The non-thermal plasma extinct when the energy input is shut down.

The present invention advantageously relies on the use of non-thermal plasma in direct contact of the object to be sterilized to achieve sterilization. This method is particularly adapted for sensitive material and complex devices. Indeed, non-thermal plasma allows sterilizing all types of material including heat sensitive polymers, resins organic materials such as cellulose, electronics, biomaterials or collagen without concern of time of exposure. The low temperature, or non-thermal plasma, allows a direct exposition of the complete device to the plasma source where the reactive spices are generated.

Moreover, non-thermal plasma lights off as soon as the energy input is turned off. After the plasma extinction the reactive species recombine in a few seconds, leaving no active principle. This is a tremendous advantage of plasma versus other sterilizers: after sterilization, the use of a non-thermal plasma thereby avoids any risks of leaving on the object sterilized any residue that could be harmful for the patient and medical staff health. Accordingly, non-thermal plasma is not only compatible with medical use, but its use is also better than hydrogen peroxide, peracetic acid or ethylene oxide.

The low temperature reached during the process is a significant advantage over heat-based sterilization process like autoclave in terms of medical device integrity preservation and the associated potential health risks.

The use of a secondary enclosure is advantageous since said secondary enclosure, enclosing the sterilized object may be transported without altering the sterile state of the object. Indeed, the secondary enclosure being impermeable to biological material, the object stays sterile once it has been sterilized by the non-thermal plasma, and while the secondary enclosure is not opened. The conservation of the sterile state of the sterilized object until it is used is a major issue for medical application. By keeping the secondary enclosure sealed and sterilized, the patient safety is enhanced.

In some embodiments, the non-thermal plasma sterilizing device further comprises a gas supply, and the secondary enclosure comprises an input port configured to be connected to the gas supply.

The overall gas input from the secondary enclosure is impermeable to biological matter to preserve the sterile state after the sterilization process.

In this configuration, the gas flow inside the secondary enclosure can be optimized to create more favorable conditions for igniting the non-thermal plasma. In particular, multiple gases may be introduced at the same time inside the secondary enclosure to optimize the sterilization. A mixture composition may evolve during the sterilization process in order to take full advantage of the gas related plasma specific properties to optimize the sterilization efficiency.

In some embodiments, the pumping system and the gas supply are configured to simultaneously:

bring the primary enclosure at a pressure below $9.10^{-4}$ bars and more preferably in range of $10^{-5}$ to $10^{-7}$ bars, and bring the secondary enclosure at a pressure at least 10 times and more preferably between 100 to 1000 times higher than the pressure in the primary enclosure.

In some embodiments, the pumping system and the gas supply are configured to simultaneously:

bring the secondary enclosure at a pressure below $10^{-2}$ bars and more preferably in range of $10^{-3}$ to $10^{-5}$ bars, and bring the primary enclosure at a pressure at least 10 times and more preferably between 100 to 1000 times lower than the pressure in the secondary enclosure.

The pressure difference between the primary enclosure and the secondary enclosure coupled to a low-pressure environment allows igniting a non-thermal plasma in the secondary enclosure. The conditions are such that the energy input to light on the plasma in the secondary enclosure is minimal, thereby minimizing the ionization fraction of the gas in order to minimize the temperature to which the device in the secondary enclosure is exposed to. For instance, in preferable conditions, coefficient $\alpha$ is below $10^{-3}$ and temperature lower than 50°C.

In a further embodiment the abovementioned pressure difference between the primary and the secondary enclosure and the low-pressure environment allow the ignition of the plasma in the secondary enclosure only. Thus, it requires less energy to ignite the non-thermal plasma in the secondary enclosure. Moreover, since the primary enclosure is free from any plasma, the device is less affected by wear and becomes more durable.

In some embodiments, the gas comprises air, argon, oxygen, carbon dioxide and/or nitrogen and mixture thereof.

Such gases are cheap, easy to access, not dangerous for users - yet in common use in hospitals for number of other applications - and do not present health risks for patients. Furthermore, these gases are not harmful for the environment, therefore the choice of these gas reduce the impact on the environment of the sterilization process.

In some embodiments, the sterilizing device comprises coils provided around the primary enclosure configured to generate a magnetic field inside the secondary enclosure.

The magnetic field allows creating a convection movement of the charged species within the ignited plasma. Said convection movement facilitates a homogenous distribution of the non-thermal plasma inside the secondary enclosure. The charge particles are accelerated by the magnetic field thereby increasing their mean-free path. At the nanoscopic level, the increase of the mean free path is directly linked to the increase of the probability of reaction each charge species before its deactivation. This means an increase of the process efficiency, kinetic and homogeneity at the macro level. Consequently, the sterilization is more homogenous and efficient inside the secondary enclosure. This step is critical to ensure a more reliable the sterilization.

In some embodiments, the device further comprises a plurality of secondary enclosures, each secondary enclosures being disposed inside the primary enclosure.

Providing multiple secondary enclosures allows sterilizing multiple objects at the same time. At an industrial scale, it represents a time economy.

In some embodiments, the electromagnetic source comprises at least one couple of antenna and mass, wherein the antenna and the mass of said couple of antenna and mass are electrically coupled, and wherein at least one secondary enclosure is provided between the antenna and the mass of said couple of antenna and mass such that the couple of antenna and mass is configured to create the electromagnetic field through the secondary enclosure.

In one embodiment theses coils may be Helmholtz coils to ensure an homogeneous field in the enclosures.

The couple of antenna and mass allows orienting the electromagnetic field through the inside of the secondary enclosure. Thus, the energy transferred to the gas encompassed inside the secondary enclosure is more precisely inputted. Thus, the ignition of the non-thermal plasma is more homogenous and requires less energy.

In some embodiments, the wherein the electromagnetic source comprises at least two couples of antenna and mass, and wherein the couples of antenna and mass are stacked in the primary enclosure in a vertical direction.

In the present description, the vertical direction corresponds to the direction of the gravity.

The stacking of couples of antenna and mass along the vertical direction is preferable. However, any type of stacking along any direction may be provided. The couples of antenna and mass may also not be stacked.

Accordingly, the electromagnetic field can be independently adapted in each secondary enclosure. Thus, this configuration is particularly advantageous when using a plurality of secondary enclosures.

In some embodiments, the secondary enclosure comprises a wall delimiting an inner volume of the secondary enclosure, the wall being permeable to electromagnetic fields and impermeable to biological material, the wall comprising a barrier portion impermeable to fluid and at least one membrane portion enabling a flow of fluid through said membrane portion.

Such a secondary enclosure allows on one hand ensuring that its inner volume stays sterile once it has been sterilized; and on the other hand, allows a gas flow to pass through the secondary enclosure without any risk of contamination by biological material. Accordingly, the secondary enclosure is particularly adapted to sterilization purposes.

The invention also relates to a method for sterilizing an object comprising:

placing an object to sterilize into a secondary enclosure, the secondary enclosure being impermeable to biological materials and permeable to electromagnetic fields;

sealing the secondary enclosure encompassing a gas;

placing the secondary enclosure into a primary enclosure of a non-thermal plasma sterilizing device;

igniting the gas into a non-thermal plasma inside the sealed secondary enclosure;

collecting the secondary enclosure containing the sterile object.

The method presents the same advantages as the sterilizing device. Said method is a quick and reliable method to sterilize an object and to keep it sterile inside the secondary enclosure during at least 30 days and more preferably at least 6 months for further use. Such a timeline complies with hospitals requirements and insures the maximum patient safety.

In some embodiments, the primary enclosure is brought to a pressure lower than $9.10^{-4}$ bars, more preferably below $10^{-5}$ bars, and even more preferably between $10^{-5}$ to $10^{-7}$ bars.

In some embodiments, the pressure in the primary enclosure is at least 10 times lower than the pressure in the secondary enclosure and more preferably 100 to 1000 times.

In such a configuration, it is possible to ignite a non-thermal plasma inside the secondary enclosure with a temperature below 50°C.

In a further embodiment, the plasma is selectively ignited in the secondary enclosure, without igniting a plasma inside the primary enclosure. Such a selective ignition is allowed by the pressure difference. Thus, it necessitates less energy to ignite the plasma because only the gas of the secondary enclosure is ignited into a plasma. In other words, the sterilization itself necessitates less energy, and is thus easier to perform at less costly.

This configuration is synergetic with a low gas pressure inside the primary enclosure. The selective ignition of a plasma inside the secondary enclosure is easier to achieve.

In some embodiments, the plasma ignition is pulsatile or discontinuous, preferably at a frequency in range of 10 to 100MHz, more preferably in range of 10 to 20 MHz and even more preferably in range of 13 to 15MHz.

In some embodiments, the non-thermal plasma is extinct for less than 5 min, preferably less than 1 min, between two consecutive ignitions.

Accordingly, these extinction phases of the plasma aim to catalyze the renewal of the atmosphere in the secondary enclosure by reintroducing species into the secondary enclosure that would be activated at the next ignition, in order to maximize the availability of reactive species in direct contact with the object.

In some embodiments, the method comprises changing the mixture of gas inside the secondary enclosure.

Multiple plasma ignition allows regenerating active species able to sterilize the object. Indeed when the plasma is ignited, it holds a wide range of unstable active species including atoms, singulets, cations, anions, metastables, astables, electrons, photons... a large part of it disappear after less than a minute as the plasma stabilize. Thus, providing repeated plasma ignitions improve the efficiency of the sterilization, which becomes quicker and more reliable.

In some embodiments, the non-thermal plasma is ignited inside the secondary enclosure during less than 5 min, preferably less than 2 min.

The time is tuned depending on the gas mixture in order to take the full advantage of the high reactivity of the plasma in the first few minutes after its ignition.

The efficiency of the method is insured by the generation of the non-thermal plasma in direct contact with all the surfaces to sterilize. This direct contact minimizes the requirement for energy and the consequent rise in temperature, it also makes the process uniquely efficient.

The inventors have observed that, once ignited, the sterilization process loose more than 65% of its efficiency within the first 5 minutes after ignition. Indeed, the most reactive species of the non-thermal plasma are consummated or deactivated within said 5 minutes. Thus, short ignition time combined with repeated ignitions quickens the method while still providing an efficient of the sterilization.

In some embodiments, during the ignition of the non-thermal plasma, the non-thermal plasma is ignited at a frequency of 100 Hz to 100 MHz, preferably in a range of 10 MHz to 100 MHz and more preferably in range of 10 MHz to 20 MHz.

While not influencing the non-thermal plasma efficiency because of the speed of execution, this pulsation reduces the load on the electromagnetic energy source and thereby the device consumption and need for maintenance.

## BRIEF DESCRIPTION OF THE DRAWINGS

**Figure 1** is a schema of a non-thermal sterilizing device according to an embodiment of the invention.

**Figure 2** is a schema of a secondary enclosure according to an embodiment of the invention.

**Figure 3** is a graphic showing the evolution of the tension necessary to ignite a gas into a non-thermal plasma versus pressure of said gas.

**Figure 4** is a flow chart showing steps of a method according to one embodiment of the invention.

## DETAILED DESCRIPTION

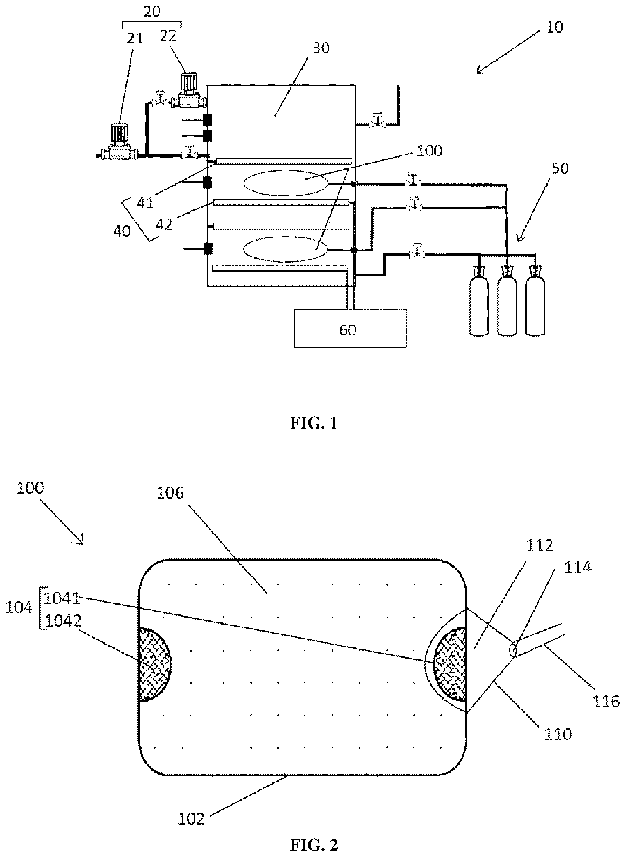

Figure 1 is a schema of a non-thermal sterilizing device 10 according to an embodiment of the invention. The sterilizing device 10 comprises a primary enclosure 30, at least one secondary enclosure 100, a pumping system 20, a gas supply 50, an electromagnetic source 60 and at least one couple of antenna and mass 40 electrically connected to the electromagnetic source 60.

It is noted that the secondary enclosure 100 can also be referred to a sterile enclosure 100. Moreover, in the present description, a sterile enclosure is an enclosure that is impermeable to biological materials. In other words, a sterile enclosure delimits an inner volume that cannot exchange biological materials with anything exterior to said inner volume. The secondary enclosure 100 is a sterile enclosure.

The secondary enclosure 100 is fluidly connected to the gas supply 50 on one hand, and to the primary enclosure 30 on the other hand. The connection to the gas supply 50 is optional. The pumping system 20 comprises a primary pump 21 and a secondary pump 22, each fluidly connected to the primary enclosure 30.

The secondary enclosure 100 is configured to encompass a gas, which may be input by the gas supply 50 or may be present at the beginning within the inner volume of the secondary enclosure 100. The pumping system 20 is configured to pump the gas encompassed inside the secondary enclosure 100. More precisely, the pumping system 20 is configured to pump the gas encompassed in the secondary enclosure 100 indirectly through the vacuum generated in the primary enclosure 30. In other words, the pumping system is configured to generate a vacuum adapted to draw gas from the secondary enclosure 100. When the sterilizing device 10 operates, a gas flow flows from the gas supply 50 to the primary enclosure 30, through the secondary enclosure 100.

The couple of antenna and mass 40 comprises an antenna 42 and a mass 41. The secondary enclosure 100 is disposed between the mass 41 and the antenna 42 of the couple of antenna and mass 40. In the illustrated embodiment, the antenna 42 and the mass 41 are stacked inside the primary enclosure 30 along the vertical direction. In embodiments where the sterilizing device 10 comprises a plurality of couples of antenna and mass 40, the plurality of couples of antenna and mass 40 may be stacked along the vertical direction, inside the primary enclosure 30. Other stacking may be considered, such as horizontal stacking.

The sterilizing device 10 may comprise coils disposed around the primary enclosure 30. The coils are configured to generate a magnetic field inside the secondary enclosure 100. The coils may be electrically connected to the electromagnetic source 60 or be connected to an independent power source.

The electromagnetic source 60 may be a standard power source, optionally connected to the standard main power. The electromagnetic source 60 may provide power to other equipment of the sterilizing device, such as display devices.

Figure 2 is a detailed schema of the secondary enclosure 100 according to an embodiment of the invention. The secondary enclosure 100 comprises a wall 102 delimiting the inner volume of the secondary enclosure 100. The wall 102 comprises a membrane portion 104 and a barrier portion 106. The wall 102 is permeable to electromagnetic fields and impermeable to biological material. Moreover, the membrane portion 104 enables flow of a fluid, for instance a gas, through said membrane portion 104 while the barrier portion 106 is impermeable to said fluid.

The wall 102 of the secondary enclosure 100 may be flexible or rigid. Is considered flexible a material that presents a Young's modulus E is typically in the range of 0,1 to 5Gpa.

$$E = \frac{\varepsilon}{\sigma} = \frac{\text{Strain}}{\text{Stress}}$$

Where:

- Stress ($\sigma$) is the force applied per unit area (e.g., in Pascals, Pa).
- Strain ($\varepsilon$) is the relative deformation, or change in length, compared to the original length (dimensionless).

A material that is not flexible is considered rigid. For instance, the barrier portion 106 of the wall 102 may be a polymer such as polyethylene, polypropylene or PVC. The membrane portion 104 may be a porous typically a non-woven material. Such a material may comprise pores sizing from 10 to 300 nm, so as to prevent biological material to pass through this material. More concretely, the Log Reduction Value [LRV] of the material is greater than 5 according to ASTM F1608. The porosity will allow a permeability to gas flowthrough which could be characterized according to ISO 5636-5 by the Berdtsen Air Permeability Method and would be typically in the range of 0.3 to 3 kPa/10 cm$^3$ . For instance, the membrane portion 104 may be Tyvek $^{©}$ or cellulose.

The membrane portion 104 is attached to the barrier portion 106. For instance, the membrane portion 104 may be heat sealed, ultrasound sealed, glued or mechanically sealed, for instance with clipses on the barrier portion 106.

Advantageously, the barrier portion 106 presents a ductile fractography. In other words, when the barrier portion 106 is torn apart, it only forms two parts without any crumbling. More particularly, the mass of the two obtained parts when the barrier portion 106 is torn apart is greater than 99,9%, preferably 99,99%, of the initial mass of the barrier portion 106.

The membrane portion 104 comprises an input membrane 1041 and an output membrane 1042. The input membrane 1041 and the output membrane 1042 are spaced apart by at least 10 cm along the wall 102. More generally, the input membrane 1041 and the output membrane 1042 may be spaced apart by at least 33% more preferably between 33% to 66% of the longest dimension of the wall 102.

The secondary enclosure 100 further comprises an injection wall 110 attached to the wall 102. The injection wall 110 surrounds the input membrane 1041 and delimits an input chamber 112. A pipe port 114 is provided on the injection wall 110 and is configured to be attached to an input pipe 116, the pipe port 114 being configured to allow a flow of gas to pass through itself, into the input chamber 112. On the other hand, the input pipe 116 is connected to the gas supply 50.

The secondary enclosure 100 comprises an opening that opens in the inner volume of the secondary enclosure 100. The secondary enclosure 100 is configured to receive an object to be sterilized inside its inner volume. The object may be put inside the secondary enclosure 100 through its opening. Once the object is placed inside the secondary enclosure 100, the opening is sealed. The sealing may be a heat seal, glueing, ultrasound sealed, mechanically sealed or any type of sealing that prevents biological material to pass through.

The gas supply 50 is configured to deliver a gas to the input chamber 112 through the input pipe 116. The gas then passes through the input membrane 1041, into the inner volume of the secondary enclosure. The permeability of the input membrane 1041 generates a pressure difference between the input chamber 112 and the inner volume of the secondary enclosure 100. When the pressure in the secondary enclosure 100 reaches below $10^{-3}$ bars enclosure, the pressure of the primary enclosure 30 is lower than 10 times, most preferably in the range of 100 to 1000 times than the pressure inside the secondary enclosure 100. Accordingly, at such a time, the pressure inside the primary enclosure 30 is below $9.10^{-4}$ bars.

In some embodiments, the secondary enclosure 100 may comprise only a pipe port 114, directly attached to the input membrane 1041.

When a gas flows through the secondary enclosure 100, a pressure difference appears between the primary enclosure 30 and the secondary enclosure 100. This pressure difference can be estimated by Darcy's law as shown in equation (1).

$$\frac{Q}{A} = \frac{k(P_2 - P_1)}{\eta . D_x} \quad : \quad (1)$$

In equation (1), Q is the gas input flow rate, A is the surface of the membrane portion 104, k is the permeability to the gas of the membrane portion 104, $P_2$ is the pressure inside the secondary enclosure 100, Pi is the pressure inside the primary enclosure 30, $\eta$ is the viscosity of the gas and $D_x$ is the thickness of the membrane portion 104.

Accordingly, it is possible to adapt the pressure difference between the primary enclosure 30 and the secondary enclosure 100 by adapting the area of the membrane portion 104 of the secondary enclosure 100.

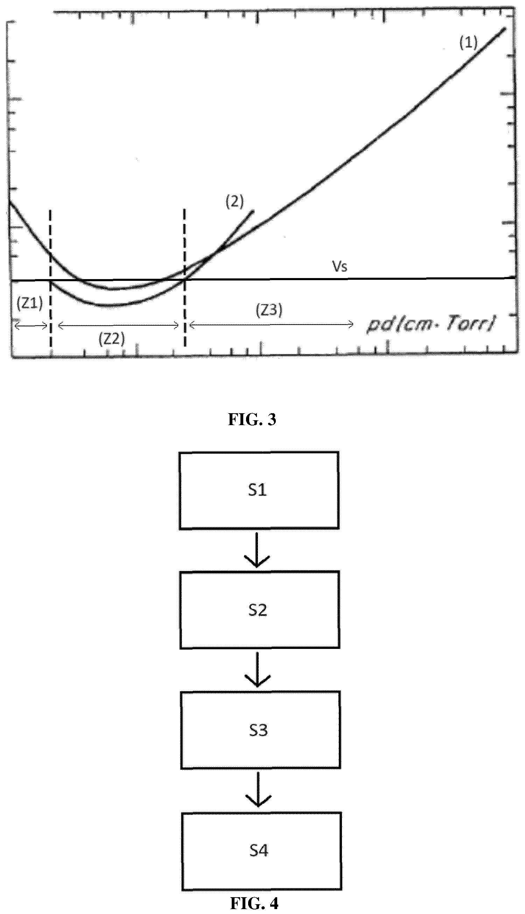

Figure 3 is a graphic showing the evolution of the tension necessary to ignite a gas into a non-thermal plasma versus pressure of said gas. These data are Paschen curves. The ignition tension has been measured for two different gases: air (1) and nitrogen (2).

As shown on figure 3, the required voltage for igniting a plasma presents a minimum. In other words, when the gas pressure is either low or high, an important voltage is required for igniting a plasma. For instance, if a tension Vs, greater than the minimal tension for igniting a plasma, is applied to a gas, three pressure zones are delimited, function of said gas pressure.

In the first zone Z1, which is the low-pressure zone, the pressure of the gas is too low for the tension Vs to ignite the plasma. In the second zone Z2, the pressure of the gas allows the plasma ignition. In the third zone Z3, the pressure of the gas is too high for the tension Vs to ignite the plasma.

In the present invention, the electromagnetic source generates a target tension Vs between the antenna 42 and the mass 41. Since the secondary enclosure 100 is disposed inside the primary enclosure 30, between the antenna 42 and the mass 41, the target tension Vs is applied to the gas encompassed inside the secondary enclosure 100. Moreover, the target tension Vs is also applied to the gas encompassed inside the primary enclosure 30, which is between the antenna 42 and the mass 41.

Accordingly, the difference in pressure between the secondary enclosure 100 and the primary enclosure 30 is adjusted so the pressure inside the primary enclosure 30 is too low for a plasma to be ignited while the pressure inside the secondary enclosure 100 allows the plasma ignition. In other words, referring to figure 3, the gas inside the primary enclosure 30 is in zone Z1 while the gas encompassed inside the secondary enclosure 100 is in zone Z2. Thus, the non-thermal plasma can be selectively ignited inside the secondary enclosure 100.

Preferably, the gas supply 50 provides air and the gas pressure inside the secondary enclosure 100 is below $10^{-2}$ bars more preferably in range of $10^{-3}$ to $10^{-5}$ bars. On the other hand, the gas pressure inside the primary enclosure 30 is below $9.10^{-4}$ bars more preferably in range of $10^{-7}$ to $10^{-5}$ bars. The gas supply may provide nitrogen, oxygen, carbon dioxide, air or argon or mixture thereof.

As explained above the pressure difference between the secondary enclosure 100 and the primary enclosure 30 is proportional to the surface of the membrane portion 104 (see equation (1)). The output membrane 1042 presents an output surface s1 and the input membrane 1041 presents an input surface $s_2$. Accordingly, the surface of the membrane portion 104 is $s_1 + s_2$. On the other hand, the barrier portion 106 presents a surface S. Accordingly, the total surface of the secondary enclosure 100 is $s_1 + s_2 + S$.

The surfaces of the membrane portion 104 and the barrier portion 106 of the secondary enclosure 100 are configured to have the ratio $\Gamma$ (2) equal or lower than 50% more preferably between 33% and 10%.

$$\Gamma = \frac{s_1 + s_2}{S + s_1 + s_2} : (2)$$

When the surfaces of the secondary enclosure 100 verify ratio $\Gamma$ (2), the gas pressure inside the secondary enclosure 100 is, according to equation (1), at least 10 times and preferably in range of 100 to 1000 times higher than the gas pressure inside the primary enclosure 30 when the pressure enclosure is established below $9.10^{-4}$ bars.

Figure 4 is a flow chart showing steps of a method according to one embodiment of the invention. The method comprises:

(S1). Placing an object to sterilize inside the secondary enclosure 100,

(S2). Placing the secondary enclosure 100 into the primary enclosure 30 of the sterilizing device 10,

(S3). Igniting a plasma inside the secondary enclosure 100,

(S4). Collecting the secondary enclosure 100 containing the sterilized object.

In step S1, the object to be sterilized is placed inside the secondary enclosure 100, through the opening of said secondary enclosure 100. Any object may be placed inside the secondary enclosure 100. However, the secondary enclosure 100 is particularly adapted to medical devices such as endoscopes. Moreover, the opening of the secondary enclosure 100 is sealed, while the secondary enclosure 100 encompasses the object to be sterilized. In addition, a gas may also be encompassed inside the secondary enclosure.

In step S2, the secondary enclosure 100 encompassing the object is placed inside the sterilizing device 10, inside the primary enclosure 30, between the couple of antenna and mass 40. The pipe port 114 of the secondary enclosure is connected to the gas supply 50 through the pipe 116.

Then, the primary enclosure 30 is closed and the pumping system 20 pumps the gas inside the primary enclosure up to a target pressure, below $9.10^{-4}$ bars, more preferably in range of $10^{-7}$ bar to $10^{-6}$ bar. On the other hand, the gas supply 50 is opened to create an airflow inside the secondary enclosure 100. The pressure in the secondary enclosure 100 is maintained in range of $10^{-5}$ bar to $10^{-4}$ bar.

In step S3, the electromagnetic source 60 is turned on, such that an electromagnetic field is generated between the antenna 42 and the mass 41 of the couple of antenna and mass 40. In particular, a target tension Vs is set between the antenna 42 and the mass 41. The target tension Vs is sufficient to ignite a non-thermal plasma inside the secondary enclosure 100 without igniting any plasma inside the primary enclosure 30.

The electromagnetic source 60 is configured to generate a pulsatile or discontinuous electromagnetic field comprising an active phase and a shutoff phase. During the active phase, the electromagnetic field sets the target tension Vs between the antenna 42 and the mass 41. During the shutoff phase, the electromagnetic field is shutdown. One active phase followed by one shutoff phase is a cycle. During step S4, at least 3 cycles are performed more preferably 10 to 15 cycles.

The active phase lasts for less than 5 minutes, preferably less than 2 minutes. The shutdown phase lasts for less than 5 minutes, preferably less than 1 minute. Accordingly, the frequency of the pulsatile electromagnetic field is in range of 10 to 100MHz more preferably 13 to 15MHz. At the end of step S4, the object disposed inside the secondary enclosure 100 is sterilized.

In step S4, the secondary enclosure 100 is removed from the sterilizing device 10, and may be stored sealed and/or transported for a further use.

## Claims

1. A non-thermal plasma sterilizing device (10) including:

   a primary enclosure (30) fluidly connected to a pumping system (20),
   a secondary enclosure (100) configured to receive an object to be sterilized, the secondary enclosure being configured to be removably disposed inside the primary enclosure (30) and comprising an output port (1042) fluidly connected to the primary enclosure (30), the secondary enclosure (100) being impermeable to biological material and permeable to electromagnetic field,
   wherein the secondary enclosure (100) encompasses a gas, the pumping system (20) being configured to pump the gas encompassed in the secondary enclosure (100) through the output port, and
   wherein the sterilizing device further comprises an electromagnetic source (60) configured to generate an electromagnetic field to ignite the gas of the secondary enclosure (100) into a non-thermal plasma.

2. The non-thermal plasma sterilizing device (10) of claim **1,** further comprising a gas supply (50), and wherein the secondary enclosure (100) comprises an input port (114) configured to be connected to the gas supply (50).

3. The non-thermal plasma sterilizing device (10) of claim **2,** wherein the pumping system and the gas supply are configured to simultaneously:

   bring the primary enclosure (30) at a pressure lower than $9.10^{-4}$ bars, and
   bring the secondary enclosure (100) at a pressure at least 10 times higher than the pressure in the primary enclosure (30).

4. The non-thermal plasma sterilizing device (10) of claim **1** to **3,** wherein the gas comprises air, argon, oxygen, carbon dioxide, and/or nitrogen.

5. The non-thermal plasma sterilizing device (10) of anyone of claims **1** to **4,** further comprising coils provided around the primary enclosure (30) and configured to generate a magnetic field inside the secondary enclosure (100).

6. The non-thermal plasma sterilizing device (10) of anyone of claims **1** to **5,** comprising a plurality of secondary enclosures (10), each secondary enclosures (100) being disposed inside the primary enclosure (30).

7. The non-thermal plasma sterilizing device (10) of anyone of claims **1** to **6,** wherein the electromagnetic source (60) comprises at least one couple of antenna and mass (40), wherein the antenna (42) and the mass (41) of said couple of antenna and mass (40) are electrically coupled, and wherein at least one secondary enclosure (100) is provided between the antenna (42) and the mass (41) of said couple of antenna and mass (40) such that the couple of antenna and mass (40) is configured to create the electromagnetic field around the secondary enclosure (100).

8. The non-thermal plasma sterilizing device (10) of claim **7,** wherein the electromagnetic source comprises at least two couples of antenna and mass (40), and wherein the couples of antenna and mass (40) are stacked in the primary enclosure in a vertical direction.

9. The non-thermal plasma sterilizing device (10) of anyone of claims **1** to **8,** wherein the secondary enclosure (100) comprises a wall (102) delimiting an inner volume of the secondary enclosure (100), the wall (102) being permeable to electromagnetic field and impermeable to biological material, the wall (102) comprising a barrier portion (106) impermeable to fluid and at least one membrane portion (104) enabling a flow of fluid through said membrane portion (104).

10. Method for sterilizing an object comprising:

placing an object to sterilize into a secondary enclosure (100), the secondary enclosure (100) being impermeable to biological materials and permeable to electromagnetic fields;
placing the secondary enclosure (100) into a primary enclosure (30) of a non-thermal plasma sterilizing device (10);
igniting the gas into a non-thermal plasma inside the sealed secondary enclosure (100) ;
collecting the secondary enclosure (100) containing the sterilized object.

11. Method according to claim **10,** wherein the primary enclosure (30) is brought to a pressure of $9.10^{-4}$ bars or lower.

12. Method according to claim **11,** wherein the pressure in the primary enclosure (30) is 100 to 1000 times lower than the pressure in the second enclosure (100).

13. Method according to anyone of claims **10** to **12,** wherein the non-thermal plasma ignition is pulsatile, preferably at a frequency in range of 10 to 100MHz, preferably in range of 10 to 20 MHz.

14. Method according to claim **13,** wherein the non-thermal plasma is extinct for less than 5 min, preferably less than 1 min, between two consecutive ignitions.

15. Method according to anyone of claims **10** to **14,** wherein the plasma is ignited inside the secondary enclosure (100) during less than 5 min, preferably lesser than 2 min.

**FIG. 1**

**FIG. 2**

**FIG. 3**

**FIG. 4**

# EUROPEAN SEARCH REPORT

**Application Number**

EP 24 30 6541

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/230426 A1 (POPOT JEAN-MARC [FR] ET AL) 5 September 2013 (2013-09-05) | 1-6,9-12 | INV.<br>A61L2/14<br>H05H1/46 |
| Y | * paragraph:[0056], [0057], [0059], [0060], [0063], [0066], [0068], [0071], [0072], [0074], [0076]; figure: 1 * | 7,8 | |
| X | EP 4 309 680 A1 (AURORA [FR]; UNIV REIMS CHAMPAGNE ARDENNE [FR]) 24 January 2024 (2024-01-24) * paragraph: [0012], [0020], [0021], [0041], [0045], [0046], [0059], [0100], [0101], [0102]; figure: 5 * | 1,2,4,6, 10,13-15 | |
| Y | WO 2013/101673 A1 (FRANKLIN MARK A [US]; FRANKLIN WILLIAM J [US]) 4 July 2013 (2013-07-04) * page 10: line 8-16; page 12: line 21-26; page 13: line 24-30; page 22: line 32; page 23: line 4-6; figure: 1 * | 7,8 | TECHNICAL FIELDS SEARCHED (IPC)<br><br>A61L<br>H05H |

The present search report has been drawn up for all claims

| Place of search | Date of completion of the search | Examiner |
|---|---|---|
| Munich | 16 April 2025 | Nania, Manuela |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...................................................................................

& : member of the same patent family, corresponding
document

Europäisches
Patentamt

European
Patent Office

Office européen
des brevets

**Application Number**

**EP 24 30 6541**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

   see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

EP 24 30 6541

The Search Division considers that the present European patent application does not comply with the requirements of unity of invention and relates to several inventions or groups of inventions, namely:

1. claims: 2-6, 9-15(completely); 1(partially)

   device comprising a primary enclosure fluidly connected with a pump and a secondary enclosure removably received therein and encompassing a gas as well as an output port, the device further comprising an electromagnetic source to ignite the gas into a non-thermal plasma, wherein the pressure in the secondary enclosure is configured to be at least 10 times higher than the pressure in the primary enclosure which is lower than 9 x 10-4 bars
   ---

2. claims: 7, 8(completely); 1(partially)

   device comprising a primary enclosure fluidly connected with a pump and a secondary enclosure removably received therein and encompassing a gas as well as an output port, the device further comprising an electromagnetic source to ignite the gas into a non-thermal plasma, wherein the electromagnetic source comprises an antenna and a mass electrically coupled
   ---

## ANNEX TO THE EUROPEAN SEARCH REPORT
## ON EUROPEAN PATENT APPLICATION NO.

EP 24 30 6541

This annex lists the patent family members relating to the patent documents cited in  the above-mentioned European search report.
The members are as contained in the European Patent Office EDP file on
The European Patent Office is in no way liable for these particulars which are merely given for the purpose of information.

16-04-2025

| Patent document cited in search report | | Publication date | Patent family member(s) | | Publication date |
|---|---|---|---|---|---|
| US 2013230426 | A1 | 05-09-2013 | CA | 2819374 A1 | 29-03-2012 |
| | | | EP | 2618851 A1 | 31-07-2013 |
| | | | ES | 2515491 T3 | 29-10-2014 |
| | | | FR | 2965179 A1 | 30-03-2012 |
| | | | US | 2013230426 A1 | 05-09-2013 |
| | | | WO | 2012038669 A1 | 29-03-2012 |
| EP 4309680 | A1 | 24-01-2024 | NONE | | |
| WO 2013101673 | A1 | 04-07-2013 | NONE | | |

For more details about this annex : see Official Journal of the European Patent Office, No. 12/82